Europäisches Patentamt

(19) European Patent Office

Office européen des brevets

(11) Publication number: **0 202 917**
**A2**

# (12) EUROPEAN PATENT APPLICATION

(21) Application number: 86303824.6

(22) Date of filing: 20.05.86

(51) Int. Cl.⁴: **A 61 F 2/02**

(30) Priority: 23.05.85 JP 111260/85

(43) Date of publication of application:
26.11.86 Bulletin 86/48

(84) Designated Contracting States:
DE FR GB IT

(71) Applicant: KUREHA KAGAKU KOGYO KABUSHIKI
KAISHA
9-11 Horidome-cho 1-chome Nihonbashi Chuo-ku
Tokyo(JP)

(72) Inventor: Takabe, Reiho
1585 Shukugawara Tama-ku
Kawasaki-shi Kanagawa-ken(JP)

(74) Representative: Perry, Robert Edward et al,
GILL JENNINGS & EVERY 53-64 Chancery Lane
London WC2A 1HN(GB)

(54) Implant device.

(57) An implant device, for transcutaneous or topical use in surgery or therapy, which comprises a moulded body of a biocompatible plastics material (e.g. a catheter 1) and, on a part of the surface of the moulded body otherwise intended to come into contact with the skin, an aggregate 5 of fibres of natural and/or synthetic polymer. The illustrated catheter 1 includes cuffs 6 and is connected to a dialysis liquid container 2 via tubing 3 and joints 4.

IMPLANT DEVICE

The present invention relates to implant devices, i.e. devices intended to penetrate the skin, such as shunts and catheters.

Biocompatible materials have contributed greatly to the development of artificial organs and implant devices, in recent years. Much work has gone into the development of materials in devices implanted or otherwise applied percutaneously, e.g. for haemodialysis, in semi-permanent catheters for continuous ambulatory peritoneal dialysis (CAPD), and for electrical leads for supplying an implanted artificial organ or receiving signals from the body. In all these contexts, it is necessary to transport energy, materials and/or signals through the skin.

An implant material, of which the skin-contacting surface comprises porous polytetrafluoroethylene (PTFE), is known. However, PTFE is hydrophobic. In addition, its surface is smooth and its porosity generally low, so that skin tissue penetrates and adheres to it only poorly. If a more porous material is used, its mechanical strength is usually low, thus providing insufficient durability for a percutaneous implant device.

EP-A-0123426 describes a flexible biocompatible material comprising a layer of a fibrous aggregate and a layer of a plastics material. The biocompatible and heat-resistant fibres which are used are, for example, of carbon, graphite, stainless steel, titanium, alumina or boron. A disadvantage of such biocompatible materials is their high production cost.

According to the present invention, an implant device comprises a moulded body of a biocompatible plastics material and, on part of the surface of the moulded body, a layer of a fibrous material. The moulded body is intended to penetrate the skin, in use, so that the fibrous material is left in contact with the skin.

The fibres are of natural and/or synthetic polymeric material. For example, naturally-occurring high polymers such as collagen, chitin and cotton, and synthetic high polymers such as polystyrene, polycarbonate, polyester, polymethyl methacrylate, cellulose acetate, nylon, polyvinyl acetate, polypropylene, polyvinylidene fluoride, hydrophilised PTFE and urethane may be used. Polyvinylidene fluoride and polyvinyl acetate are preferred. The hydrophilic polymer which is used is preferably selected on the basis that the contact angle of a droplet of octane brought into contact with the solid matter, placed in water, is less than 50°. Single or double filaments, twists, spun yarn or staple fibres may be used.

The shape and form of the fibrous layer may be selected as desired, depending on the intended use and the area of use. The fibrous material comprises mutually-intertwined fibres; they may be knitted, woven, non-woven or in the form of a felt or wound yarn. The fibrous material preferably has open pores from 20 to 1000 μm in size, so that tissue cells can easily enter into the aggregate.

The moulded body may be produced by, for example, injection-moulding, extrusion-moulding, pressure-moulding or vacuum-moulding. Its shape may be, for example, hollow, solid cylindrical, sheet-like, film-like or some more complex form. The shape will of course be determined according to the intended use.

The biocompatible plastics material may be conventional. Elastomers may be used. Examples of suitable biocompatible plastics materials are fluoropolymers such as PTFE, silicone resins such as silicone rubber, polyvinyl chloride, polyvinylidene chloride, fluorinated silicone rubber, polyethylene, polypropylene, polyesters, poly(hydroxyethyl methacrylate), polyacrylamide, polysulphone, poly(N-vinylpyrrolidone) and segmented polyurethanes.

It is often preferred to subject the plastics material to surface treatment, by etching and/or glow discharge, or by applying a surface-treatment agent. Further, if the moulded body is a tubular composite material for the passage of blood, it is often desirable to coat the inner surface of the tube with an anti-coagulant such as heparin, urokinase, albumin or streptokinase.

The fibrous aggregate is bonded to the moulded body by any suitable means. The porosity and flexibility of the fibrous layer should not be reduced. For example, a thin coating of an adhesive is applied to the surface of the moulded body, and the fibrous aggregate is applied to the thus-treated body under pressure. Suitable adhesives are silicones, ethylene-vinyl acetate copolymers, polyesters, nylons, urethane elastomers, polyvinyl acetate and acrylic resins. The fibrous aggregate should not be fully bonded by the adhesive, in order to retain the flexibility of the whole and the aggregate's own ability to absorb tissue cells.

Alternatively, the surface of the moulded body is heated and the fibrous aggregate bound thereto by fusion. The fibres may be applied directly, or knitted into or wound around the coated or partially-plasticised surface of the moulded body. If the components have sufficient strength, the fibres may be twisted around the moulded body, or the fibres may be bag-knitted, force then being applied to cover the plastics material with the bag-knitted fibres.

A product of the invention may include an electroconductive material such as a copper wire. The product is then appropriate for electrical input, or the output of signals from the body.

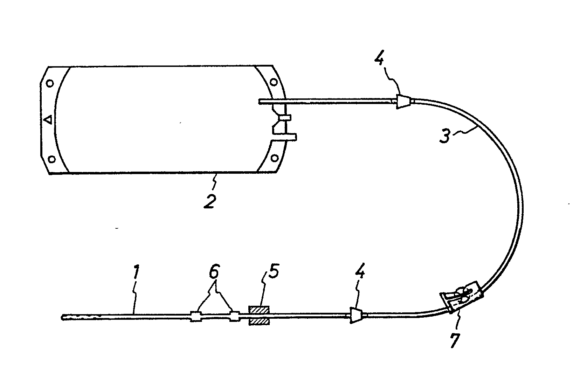

The invention will now be described by way of example only with reference to the accompanying drawing which is a schematic representation of an embodiment of the invention incorporated in apparatus for CAPD. The drawing shows a catheter 1 (e.g. of silicone) and a container 2 for dialysis liquid, connected via tubing 3 and joints 4 (e.g. of ceramic or titanium). Bag-knitted fibres 5 (e.g. of nylon) have been bound to the surface of the catheter 1 (e.g. using a silicone adhesive). The fibres 5 are at the position where the catheter penetrates the skin, in use. The apparatus also includes cuffs 6, for fixing the catheter 1 within the body, and a valve 7, for controlling fluid flow.

For the purposes of illustration, the catheter 1 is of the Tenckhoff type. The cuffs 6 are conventional but are in fact unnecessary in the context of the present invention because the fibres 5 bond sufficiently well with skin tissue. The absence of the cuffs 6 simplifies the catheter and its operation.

More generally, a product of the invention has open pores and a surface of hydrophilic fibres having a degree of unevenness specific to the fibrous aggregate, at the point of skin contact. These characteristics allow the tissue cells of a living body to penetrate the product and become biologically fixed, thereby providing good strength. Products of the invention are inexpensive. They can be used for various implant devices, e.g. the outer shunts for artificial dialysis, in electrical connections, and for trans-cutaneous connection as in CAPD. Products of the invention can be used to carry the blood or perfusate in, for example, haemodialysis, haemoperfusion, plasma exchange, plasma filtration and peritoneal dialysis, the heating medium for thermo-therapy of internal organs, or the electrical connections for driving an artificial heart or pancreas, or for recording internal or topical temperature values and internal electromotive forces.

The following Example illustrates the invention.

Example

A silicone adhesive was applied, to a thickness of 0.1-0.3 mm, on the surface of part of a silicone rubber tube (3.0 mm inner diameter, 5.0 mm outer diameter, 30 mm long). The thus-treated part of the tube was then covered with a nylon aggregate in bag form. Both ends of the aggregate were pulled, so that the aggregate contacted and adhered to the tube.

The same procedure was conducted using fibrous bags of polyvinylidene fluoride, polyvinyl acetate and polypropylene. In each case, the product retained its flexibility.

Each of the products was tested by hermetically sealing one end of the tube with a silicone adhesive, and cutting the tube to a length of 10 mm. After steam-sterilisation, the tube was implanted percutaneously into a wound caused by excision (under light anaethesia with ether) of a depilated dorsal part of a Sprague-Dawley rat (about 223 ± 12 g body weight, 10 weeks old). The closed end of the tube was in the body of the rat, and the open end outside the body.

One week after the operation, the wound had healed and the implanted tube was fixed in position. Two weeks after the operation, the tube remained implanted, and there was no infection.

The rat was sacrificed. The interface of the skin and the implant was examined, and it was observed that skin cells had entered into, and filled up, the fibrous structure.

By way of comparison, the same experiment was conducted but without using the fibrous covering. One week after the operation, the skin around the implanted tube showed infection, and the tube fell out about two weeks after the operation.

0202917

CLAIMS

1.    An implant device, for transcutaneous or topical use in surgery or therapy, which comprises a moulded body of a biocompatible plastics material and, on a part of the surface of the moulded body otherwise intended to come into contact with the skin, an aggregate of fibres characterised by being of natural and/or synthetic polymer.

2.    A device according to claim 1, wherein the natural and/or synthetic polymer is selected from collagen, chitin, polystyrene, polyesters, polycarbonates, polymethyl methacrylate, cellulose acetate, nylons, polyvinyl acetate, polypropylene, polyvinylidene fluoride and polyvinyl acetate.

3.    A device according to claim 1 or claim 2, wherein the moulded body is a tube.